# EUROPEAN PATENT APPLICATION

(11) **EP 4 414 927 A1**
(43) Date of publication of application: **14.08.2024**
(21) Application number: 23155601.0
(22) Date of filing: 08.02.2023
(51) Int. Cl.: G06Q 50/22, G16H 40/20

(54) **METHOD AND SYSTEM FOR PROVIDING HEALTHCARE SUPPORT**

(71) Applicant: Koninklijke Philips N.V., 5656 AG Eindhoven (NL)
(72) Inventor: WINTER, Stefan, Eindhoven (NL); VAN GENUGTEN, Lenneke, Eindhoven (NL); PAUWS, Steffen Clarence, 5656AG Eindhoven (NL)
(74) Representative: Philips Intellectual Property & Standards

(57) **Abstract**

The present invention relates to computer implemented methods and systems for providing healthcare support. The methods include receiving data from a patient and/or a caregiver(s) and determining, based on the received data, whether an interaction between the caregiver and the patient is occurring, or when a potential interaction between the caregiver and the patient may occur. In response to determining that the interaction between the caregiver and the patient is occurring, or when a potential interaction between the caregiver and the patient may occur, a support option from a plurality of support options is determined. The caregiver is prompted to perform the determined support option.

## Description

### FIELD OF THE INVENTION

This invention relates to systems and methods for providing healthcare support, and in particular systems and methods for facilitating the provision of healthcare support to a patient by a caregiver.

### BACKGROUND OF THE INVENTION

Informal care (e.g. care from family members, neighbors and friends) provides an important role in the support of patients alongside or as an alternative to formal care (e.g. care from health professionals and employed carers). This informal care is especially important for enabling patients based outside of hospitals and other care settings to live independently.

For patients with long term health conditions, informal care can affect how patients experience their condition. For example, for patients with chronic obstructive pulmonary disease (COPD) it has been shown that informal care facilitates improved mental health outcomes in particular. It has also been shown that informal care is associated with improved health outcomes for patients with cardiovascular disease.

Informal care can be a more cost-effective way to provide support to patients compared to formal care. However, it can be more challenging for informal caregivers to support patients in a way that leads to improved health outcomes for the patient, since they generally lack the experience and skills of formal caregivers. Furthermore, informal caregiving is often a long-term task with a negative impact on the stress and wellbeing of the caregivers. Accordingly, there is a need to provide systems and methods for providing healthcare support from informal caregivers to a patient.

### SUMMARY OF THE INVENTION

The invention is defined by the claims.

According to examples in accordance with an aspect of the invention, there is provided a computer implemented method for providing healthcare support, wherein the method comprises carrying out a healthcare support process comprising the steps of:
receiving data from a patient and/or a caregiver;
determining, based on the received data, whether an interaction between the caregiver and the patient is occurring, or when a potential interaction between the caregiver and the patient may occur;
in response to determining that the interaction between the caregiver and the patient is occurring, or when a potential interaction between the caregiver and the patient may occur,
determining a support option from a plurality of support options, and
prompting the caregiver to perform the determined support option.

The detection of an interaction or the prediction of a potential future interaction between a caregiver and patient triggers the determination of an appropriate support option and the subsequent prompting for the caregiver to provide that support option. This facilitates improved patient care in terms of improved health outcomes whilst enabling efficient resource usage. In particular, by prompting the caregiver to provide support in response to the detection of a natural/random interaction the caregiver is triggered to provide support at a relevant time. Natural/random interactions are therefore more likely to be used to provide health-related support to the patient. Thereby, improved health outcomes for the patient with reduced processing resource requirements are facilitated.

In some embodiments, the healthcare support process further comprises:
collecting data from the patient using a patient sensor array, and/or
collecting data from the caregiver using a caregiver sensor array.

A sensor array may comprise one sensor, or multiple sensors. Sensors can be used to provide automatic and unobtrusive data collection from the patient and/or the caregiver. That is, it is possible to gather information relevant to current and future interactions without the need for additional input from either party. This has the advantage of convenience for both the caregiver and patient.

In embodiments involving collecting data from a patient and/or caregiver sensor array, the patient sensor array and/or caregiver sensor array may be configured to detect communication between the patient and the caregiver,
wherein the step of determining whether interaction between the caregiver and patient is occurring, or when a potential interaction between the caregiver and patient may occur comprises processing the detected communication.

By detecting communication between the patient and the caregiver using a sensor/sensors it is possible to gather information relevant to current and future patient-caregiver interactions. Processing the detected communication may involve using voice recognition to determine that the caregiver and patient are currently together. The sensor array may comprise a microphone, for example a microphone within a mobile phone device belonging to the patient or caregiver. Optionally, the sensor array may be configured to detect and collect audio from a phone conversation between the patient and process that information to determine when a potential interaction may occur. Similar information can be extracted by detecting and processing written communication between the patient and caregiver, such as an SMS message or email.

In embodiments involving collecting data from a patient and/or caregiver sensor array, the patient sensor array and/or caregiver sensor array may comprise a location sensor, and
wherein the step of determining whether interaction between the caregiver and patient is occurring, or when a potential interaction between the caregiver and the patient may occur comprises processing location data received from the location sensor to determine the location of the caregiver and/or patient.

The inventors have realized that the location of either or both parties can be used to accurately predict whether an interaction is about to occur or is occurring in a convenient way. The location sensor may be a GPS sensor in a mobile phone device belonging to the patient or caregiver. Where the method uses a sensor having another purpose (such as a sensor included in a mobile phone) this has the advantage of reduced expense and convenience for the caregiver/patient as they do not need to purchase and carry a separate device.

In some embodiments, the data from the patient and/or the caregiver comprises scheduling data, and
wherein the step of determining whether interaction between the caregiver and the patient is occurring, or when a potential interaction between the caregiver and the patient may occur comprises processing the scheduling data received from the patient and/or the caregiver to identify a scheduled meeting between the patient and the caregiver.

Scheduling data comprises information relating to the patient's and/or the caregiver's schedule. Processing information relating to the patient's and/or caregiver's schedule can provide an accurate and unambiguous indication of an intention for the parties to meet. For example, the method may involve extracting meeting information from the patient's and/or caregiver's calendar.

In some embodiments, the healthcare support process further comprises:
collecting data from the patient using a patient sensor,
collecting data from the caregiver using a caregiver sensor,
determining the distance between the patient and the caregiver based on the data from the patient sensor and the caregiver sensor, and
wherein the step of determining whether interaction between the caregiver and the patient is occurring, or when the next interaction between the caregiver and the patient will occur uses the distance between the patient and the caregiver as an input.

The patient sensor may be part of a patient sensor array. The caregiver sensor may be part of a caregiver sensor array. The inventors have realized that the distance between the parties provides a strong indication of whether an interaction is occurring or about to occur. Both the patient sensor and caregiver sensors may be location sensors, such as GPS sensors. Alternatively, the sensors may be Bluetooth sensors and the step of determining the distance between the patient and caregiver may involve using the strength of the Bluetooth connection between the two sensors.

The method may further comprise:
determining whether a user is authorized to act as a caregiver to the patient, and in response to determining that the user is authorized to act as a caregiver, carrying out the healthcare support process of any preceding claim, and optionally
receiving an access code as an input, and determining whether the user is authorized to act as a caregiver to the patient based on the input.

By including an authorization step for verifying whether a caregiver is authorized to support a patient improved security is provided. This helps to protect the patient's information from misuse and protects the patient's safety. The access code may be a barcode, more specifically a QR code. The access code may also be a URL address.

In some embodiments, determining a support option from the plurality of support options comprises processing data received from the patient and/or the caregiver to extract contextual information about the current or potential interaction and using the contextual information to determine a preferred support option, and
wherein prompting the caregiver to perform the determined support option comprises prompting the caregiver to perform the preferred support option.

By prompting the caregiver to perform a preferred option determined using contextual information the patient can receive the most relevant and appropriate care. Thereby enabling better health outcomes for the patient. The contextual information may include the relative locations of the parties. For example, if the patient and caregiver are interacting over the phone rather than in person, providing a medication reminder rather than administrating medication in person may be a more appropriate support option. The contextual information may include how long the potential interaction is expected to be. For example, a preferred support option could be determined based on how long the support option will take to complete relative to the length of the interaction. The contextual data may include information regarding the urgency of at least some of the support options. The preferred support option may be determined based on which support option is most urgent.

In some embodiments, the healthcare support process further comprises:
after prompting the caregiver to perform the determined support option, receiving a user input relating to an additional support option, and
adding the additional support option to the plurality of support options.

Providing a method implementing user input to add further support options provides a more flexible system since it is possible for additional options to be added by a user. In particular, the support options can be updated so that the available support choices for future interactions can be informed by recent interactions. Thereby, the support options stay relevant and the health outcomes for the patient are improved.

In some embodiments, the healthcare support process further comprises:
receiving user feedback from the patient and/or caregiver as an input,
storing the user feedback, and
optimizing the support provided based on the stored feedback.

Providing a method implementing user input to add user makes it possible for improved care to be provided since information from the feedback is available. Optionally, the stored user feedback can be used to optimize future interactions. For example, the feedback may indicate that a patient dislikes a specific support option being performed. In response to this feedback, this support option can be deprioritized compared to other more preferred support options.

In some embodiments, the method for providing healthcare support is a method for providing medical support, wherein the healthcare support process is a medical support process and the support options are medical support options.

Medical support options include providing medication reminders and/or assistance, vital-sign monitoring (e.g. blood pressure, temperature, pulse rate and/or respiratory rate), analyte monitoring (e.g. oxygen and/or glucose), nutritional assistance and/or hydration assistance.

In some embodiments, the healthcare support process further comprises:
detecting that the support option has been provided,
in response to detecting that the support has been provided, removing the support option from the plurality of support options and optionally wherein detecting that the support has been provided is based on a user input.

By removing completed support options, support can be provided to the patient more efficiently since support options which have already been completed will not be suggested. The facilitates better health outcomes for the patient.

In some embodiments, the healthcare support process further comprises:
receiving data from multiple caregivers,
determining, based on the received data, whether an interaction between each caregiver and the patient is occurring, or when a potential interaction between each caregiver and the patient may occur;
in response to determining that multiple interactions are occurring or may occur between the patient and different caregivers over a predetermined time period, determining a preferred caregiver for providing a determined support option,
wherein prompting the caregiver to perform the determined support option comprises prompting the preferred caregiver to perform the determined support option.
and optionally wherein determining the preferred caregiver comprises processing stored data relating to the availability and skills of each caregiver.

The inventors have realized that improved quality of care can be provided in cases where multiple caregivers are involved by including a step for determining the most appropriate caregiver for providing support. This better quality of care enables better health outcomes for the patient. The caregiver is one of the multiple caregivers.

In some embodiments, the computer implemented method further comprises sending an update to the plurality of caregivers regarding the support options which have been performed. Where multiple caregivers are involved, the stress of the caregivers can be reduced by providing updates on the care provided.

In some embodiments, prompting the caregiver to perform the determined support option comprises triggering a notification on a personal communication device, and/or an audio alert, and/or a visual alert.

Providing a method comprising triggering a notification on the caregiver's personal communication device makes it more convenient for the caregiver to receive prompts and thereby facilitates improved patient care.

According to examples in accordance with another aspect of the invention, there is provided a system for implementing the computer implemented method of any method described herein, the system comprising:
a processor configured to carry out the method of any preceding claim.

According to examples in accordance with another aspect of the invention, there is provided a computer program product comprising instructions which, when the program is executed by a computer, cause the computer to carry out the steps of any computer implemented method described herein.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of a computer implemented method for providing healthcare support, according to an embodiment of the invention.
Fig. 2 shows another example of a computer implemented method for providing healthcare support, according to an embodiment of the invention.
Fig. 3 shows another example of a computer implemented method for providing healthcare support, according to an embodiment of the invention.
Fig. 4 shows an example of a system for implementing a computer implemented method for providing healthcare support, according to an embodiment of the invention.
Fig. 5 shows another example of a system for implementing a computer implemented method for providing healthcare support, according to an embodiment of the invention.
Fig. 6 shows another example of a computer implemented method for providing healthcare support, according to an embodiment of the invention.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a computer implemented method for providing healthcare support. The method comprises receiving data from a patient and/or a caregiver and determining, based on the received data, whether an interaction between the caregiver and the patient is occurring, or when a potential interaction between the caregiver and the patient may occur. In response to determining that the interaction between the caregiver and the patient is occurring, or when a potential interaction between the caregiver and the patient may occur, a support option from a plurality of support options is determined. The caregiver is then prompted to perform the determined support option.

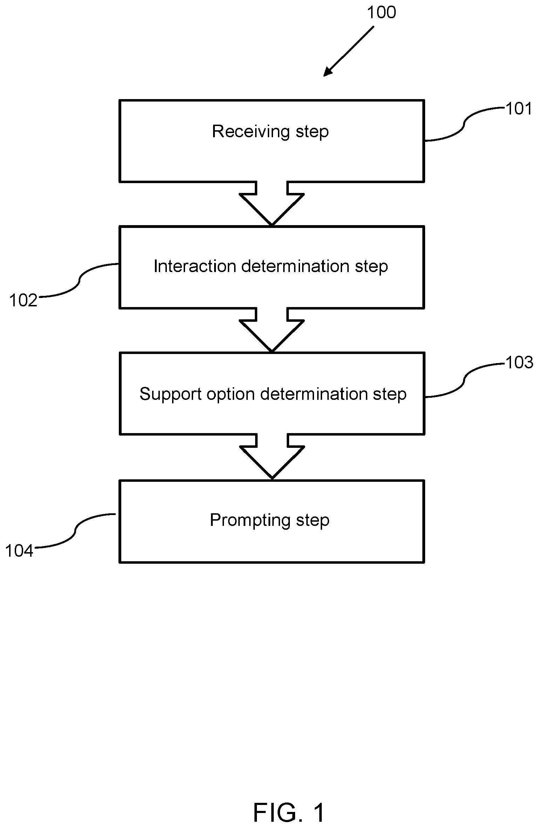

Fig. 1 shows an example of a computer implemented method 100 for providing healthcare support to a patient from a caregiver. The method includes a receiving step 101 wherein data is received from a patient and/or a caregiver. In an interaction determination step 102, it is determined whether an interaction between the patient is occurring, or when a potential interaction between the caregiver and the patient may occur, based on the data received during the receiving step 101. In response to determining that the interaction between the caregiver and the patient is occurring, or when a potential interaction between the caregiver and the patient may occur, a support option determination step 103 is carried out. The support option determination step 103 involves determining a support option from a plurality of support options. The prompting step 104 involves prompting the caregiver to perform the determined support option.

In some embodiments the interaction determination step 102 involves determining whether an interaction between the patient and the caregiver is occurring in response to receiving data indicating that the patient and caregiver are currently communicating with each other. This determination may involve monitoring phone activity and/or processing received audio data - for example, by using a voice recognition algorithm.

In some other embodiments, the interaction determination step 102 involves determining that a potential future interaction may occur and predicting when that potential future interaction will take place. This determination may involve processing the received data using a prediction algorithm configured to determine the occurrence and timing of a potential future interaction based on the received data and/or extracting key information from the received data.

The plurality of support options may include medical support tasks such as: providing medication reminders and/or assistance, vital-sign monitoring (e.g. blood pressure, temperature, pulse rate and/or respiratory rate), analyte monitoring (e.g. oxygen and/or glucose) nutritional assistance and/or hydration assistance. The plurality of support options may include other types of healthcare support such as personal hygiene assistance, assistance with attending medical appointments, provision of social support (e.g. playing a game), provision of cognitive support (e.g. filing in forms) and/or provision of emotional support.

In some embodiments, the data from the patient and/or the caregiver comprises scheduling data (e.g. calendar entries). The scheduling data may indicate that a meeting between the patient and caregiver is scheduled (and the data may be obtained from at least one of their calendars) - thereby indicating that a future meeting will occur. In another embodiment, the scheduling data may comprise navigation data - e.g., the destination in the patient and/or caregivers navigation system. For example, if the destination of the caregiver's navigation system is set to the patient's residence this may indicate that the parties intend to meet.

In some embodiments, the receiving step 101 involves receiving data collected from a patient sensor array and/or a caregiver sensor array. The patient sensor array and/or the caregiver sensor array may comprise one or more sensors.

At least one of either the patient or caregiver sensor arrays may be configured to detect communication between the patient and the caregiver. For example, one of the sensor arrays may be configured to detect a conversation (such a mobile phone conversation) between the patient and the caregiver using a microphone and/or a mobile phone application. The interaction determination step 102 may involve determining that a remote interaction, rather than an in-person interaction, between the patient and caregiver is occurring (e.g. a phone conversation). In embodiments in which the determining step comprises determining that a potential future interaction may occur, the interaction determination step 102 may comprise analyzing the audio from that phone conversation to determine a potential future interaction. Alternatively, the sensor arrays may be configured to detect an in-person conversation between the patient and the caregiver using a microphone and/or a mobile phone application. The interaction determination step 102 may then comprise determining that an interaction is currently occurring.

In some embodiments involving a patient sensor array and/or caregiver sensor array, either or both arrays may comprise a location sensor - such as a GPS sensor. For example, if the caregiver sensor array comprises a location sensor, the interaction determination step 102 may determine that an interaction is occurring if the received data indicates that the caregiver is at the patient's place of residence. If both the patient and caregiver sensor arrays comprise a location sensor, it can be accurately determined whether they are in the same place and accordingly whether an interaction is occurring.

In some embodiments, the receiving step 101 may involve receiving data collected from a patient sensor device and a caregiver sensor device. At least one of the patient and caregiver sensor devices may be a GPS sensor, Bluetooth sensor, or Wi-Fi sensor (for example). Both of the patient and caregiver sensor devices may be the same type of sensor. Alternatively, the sensors of the patient sensor device and the caregiver sensor device may be different types of sensors.

The distance between the patient and the caregiver may be calculated using the data received from these sensors. For example, in the case where both sensors are Bluetooth sensors, the distance may be determined based on the strength of the Bluetooth connection. In cases where both sensors are Wi-Fi sensors, the distance may be determined based on both sensors being connected to the same Wi-Fi connection point. The distance may be a general indication of the proximity of the patient and the caregiver rather than a precisely measured distance. For example, in the case where both sensors are Wi-Fi sensors, if both sensors are connected to the same Wi-Fi point, the distance between the patient and caregiver may be categorized as close enough to indicate an interaction. The interaction determination step 102 may involve using the distance estimated as an input to facilitate determination of whether an interaction is occurring or may potentially occur.

In some embodiments, the support option determination step 103 involves determining contextual information about the current or potential interaction and using the contextual information to determine a preferred support option. For example, the contextual information may comprise information indicating whether the interaction is remote or in-person. Different support options may be appropriate if a remote interaction is determined compared to an in-person interaction - e.g., emotional support may be a preferred support option if a remote interaction is detected, whereas some support options, such as administrating medication, will only be possible during in-person interactions. Contextual information may comprise information indicating the urgency of some or all of the support options. For example, the contextual information may include information relating to medication timings. If a certain medication is due or overdue around the time of an interaction, reminding or helping the patient to take the medication may be the preferred support option. The support option determination step 103 may involve taking into account more than one type of contextual information when determining the preferred support option - for example, whether the interaction is remote in addition to whether medication is due or overdue. The contextual information may also comprise an indication of the patient's status - for example, their levels of anxiety, loneliness, heart rate and/or dyspnea. This information may be obtained using patient surveys or sensors. In some embodiments, the support option contextual information includes an identified patient need. The patient need may be identified using sensor data, scheduling data (i.e., calendar entries) or a user input from the patient. For example, data from an analyte monitor may indicate that the patient has a need for assistance with the administration of medication. In some embodiments, scheduling data can be used to identify a future medical appointment which is turn can be used to identify a patient need for the caregiver to accompany the patient to the hospital. Patient needs may also be determined by accessing and extracting information from the patient's electronic medical record.

The prompting step 104 may involve triggering a notification on a personal communication device. For example, the caregiver may receive a text message or audio alert on a personal communication device. In some embodiments, the notification may be a vibration alert, an audio alert and/or a visual alert. If a potential future interaction is determined, the time between prompting the caregiver to perform the support option and the caregiver reaching the patient can be used to provide the caregiver with additional relevant information. For example, the computer implemented method may further comprise playing an audio message for the caregiver to listen to as they are travelling to the patient.

In some embodiments, the method may further comprise a step of receiving a user input relating to an additional support option and adding the additional support option to the plurality of support options. For example, following an interaction, the caregiver may notice that the patient appears to be suffering from low mood or anxiety and accordingly add a support option for providing emotional support to the plurality of support options. In some embodiments, the user may also add notes to provide further information relating to the support options or status of the patient. This information may be used later to inform future support options, thereby facilitating improved provision of patient care since additional relevant information is available and can be taken into account.

The inventors have realized that by providing a computer implemented method according to the embodiment shown in Fig. 1, that it is possible to provide improved patient care in terms of improved health outcomes whilst enabling efficient resource usage.

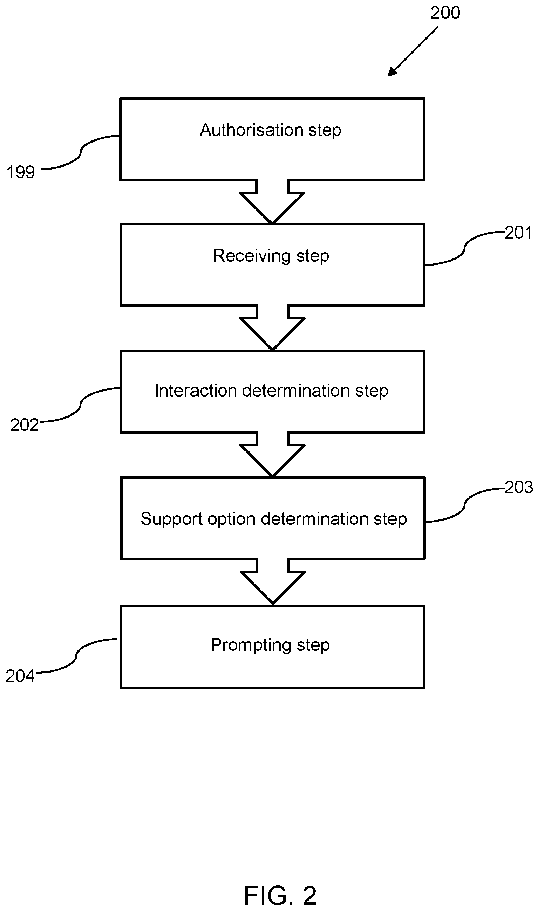

Fig. 2 shows another example of a computer implemented method 200 for providing healthcare support to a patient from a caregiver. The method includes a receiving step 201, an interaction determination step 202, a support option determination step 203 and a prompting step 204. The method further includes an authorization step 199 for determining whether a user is authorized to act as a caregiver to the patient. If it is determined that the user is authorized to act as a caregiver, the other steps of the computer implemented method can be carried out. If, instead, it is determined that the user is not authorized to act as a caregiver the other steps of the method will be aborted. That is, data will not be received from the user and/or the patient, a support option will not be determined and the user will not be prompted to support the patient.

In some embodiments, the user will be able to access relevant information about the patient after it has been determined that they are authorized to act as a caregiver. Whereas the user will also not be able to access relevant information about the patient if they have not been successfully authorized.

In some embodiments, the authorization step 199 may comprise receiving an access code as an input, and determining whether the user is authorized to act as a caregiver to the patient based on the input. The access code may facilitate an automatic determination of whether the user is authorized to act as a caregiver. This improves patient safety by providing enhanced security. The access code may, for example, be a barcode (e.g. a QR code) or a URL address. If the access code is a barcode, the user may provide the access code as an input to the method by scanning it using a personal communication device. If the access code is a URL address, the user may provide the access code as an input to the method by entering the URL address into a web browser.

In some embodiments, the access code is distributed to trusted individuals (e.g., by the patient or a formal caregiver). The access code could also be provided by confirmation from the patient that the user is authorized to support them. This confirmation could be provided during an in-person visit, using the patient's personal communication device (e.g., by inputting a confirmation to a smartphone device) and/or during a video call, for example.

In some embodiments, the authorization of a user (i.e., potential caregiver) will only be possible if the user is determined to be in the patient's home. This determination may be based on data from location sensors. For example, in embodiments where authorization of a user is achieved using an input to the patient's personal communication device, the method may involve a determination of whether the user is in the patient's home/care setting. In these embodiments, if it is determined that the user is in the patient's home/care setting then an input confirming the authorization to the patient's personal communication device will be accepted and the user will be authorized to act as a caregiver. If it is determined that the user is not in the patient's home/care setting then an input confirming the authorization to the patient's personal communication device will not be accepted and the user will not be authorized to act as a caregiver. This provides enhanced security.

In some embodiments, the computer implemented method involves receiving a video input. For example, the patient may authorize a user by holding a printed barcode in front of a camera during a live video session to authorize that user as a caregiver. In some embodiments, the user may also be prompted to present a barcode to a camera to confirm their identity as part of the authorization step.

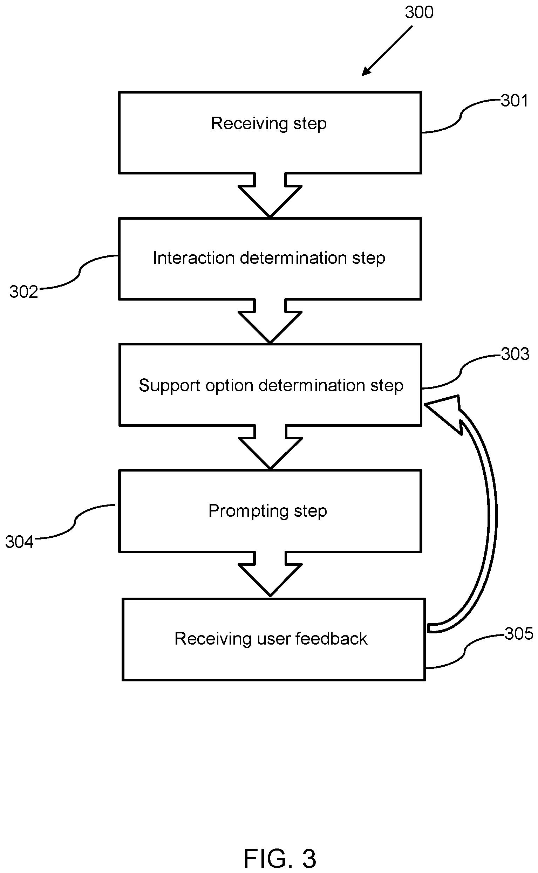

Fig. 3 shows another example of a computer implemented method 300 for providing healthcare support to a patient from a caregiver. The method includes a receiving step 301, an interaction determination step 302, a support option determination step 303 and a prompting step 304. The method further includes a step for receiving user feedback 305. The user feedback is provided via a user input, e.g., a graphical user interface. This feedback may be from the patient and/or the caregiver. Either party may want to give feedback on the system - e.g., patient satisfaction, provider stress, conveniency, etc. This information can be used to optimize future interactions. For example, the information may include an indication of how much the patient appreciated the caregiver performing a specific support option. If the information indicates that the patient did not appreciate a particular support option being performed, this support option may not be suggested in future. The method may involve classifying the support options in terms of patient preference, and prompting the caregiver to perform the patient's preferred support options first so that these are prioritized.

In some embodiments, the computer implemented method may further comprise removing a support option from the plurality of support options when it has been completed. The completion of the support option may be detected automatically (e.g. by analyzing a phone conversation between the patient and the caregiver, and/or using a sensor) or in response to a user input. Once the completion of the support option has been detected, the support option may be removed from the plurality of support options. Support options may also be removed after a predetermined time (i.e., after they are no longer relevant).

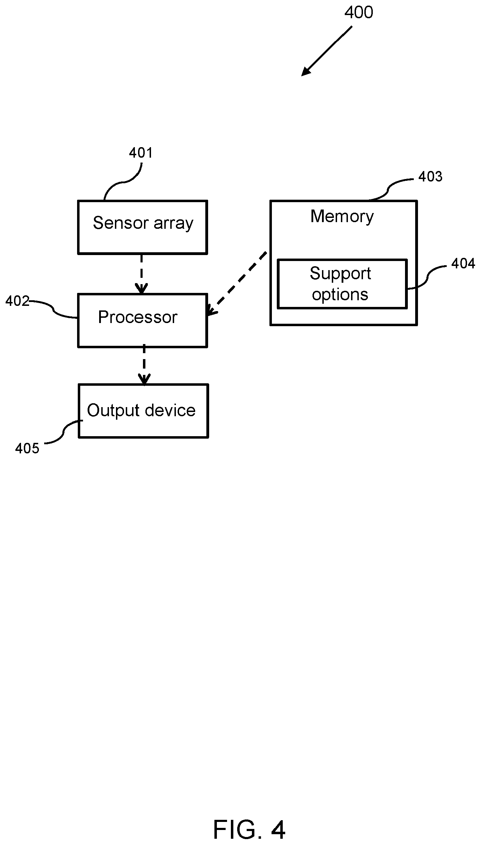

Fig. 4 shows an example of a system 400 for carrying out a computer implemented method for providing healthcare support. In this embodiment, the system includes a sensor array 401, a processor 402, an output device 405 and a memory 403 which stores a plurality of support options 404. The sensor array 401 is for collecting data from either the patient or the caregiver. The processor 402 is for carrying out the step of determining, based on the data received from the sensor array 401, whether an interaction between the caregiver and the patient is occurring, or when a potential interaction between the caregiver and the patient may occur. The processor 402 is also configured to determine a support option from the plurality of support options 404 stored in the memory 403. The output device 405 is configured to prompt the caregiver to perform the support option. In some embodiments the output device may include a personal communication device for prompting the caregiver to carry out a support option.

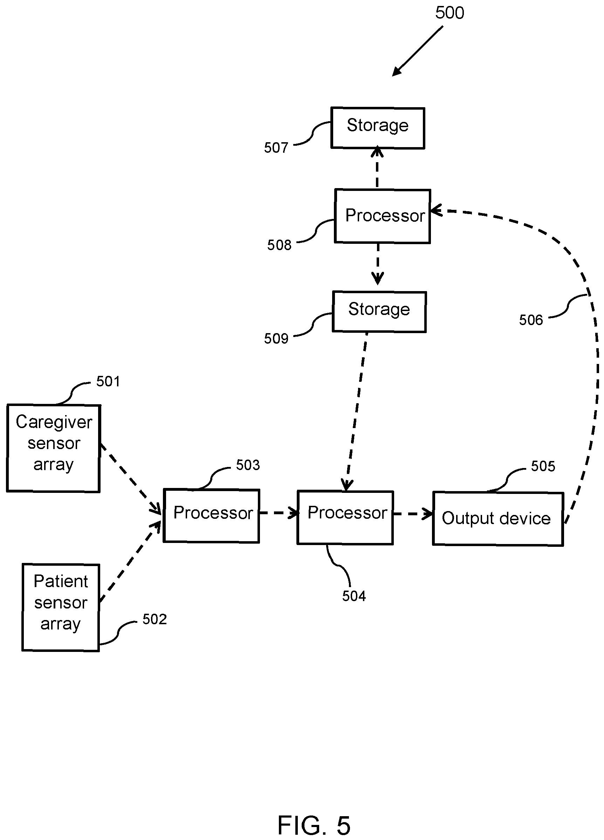

Fig. 5 shows another example of a system 500 for carrying out a computer implemented method for providing healthcare support. In this embodiment, the system includes a caregiver sensor array 501 and a patient sensor array 502. The caregiver sensor array 501 and patient sensor array 502 include sensors for collecting information about the caregiver and patient, respectively. For example, each or both sensor arrays may include location sensors and/or sensors for detecting communication between the patient and the caregiver. The system also includes a first processor 503 configured to receive, combine and analyze the data from the caregiver sensor array 501 and patient sensor array 502. The first processor 503 is configured to determine whether an interaction is occurring or when a potential interaction may occur. The system includes a second processor 504 for determining the most appropriate support option in response to the determination by the first processor 503. The system further includes an output device 505 for prompting the caregiver to provide the determined support option. In some embodiments, the output device 505 may also provide (e.g. via display text) additional information relating to the support options, and/or patient status, to facilitate the provision of improved care.

In the embodiment shown in Fig. 5, the output device 505 is configured to receive user feedback. This may be from the patient, the caregiver, or both parties. This feedback 506 is used as an input to a third processor 508. The third processor 508 is configured to receive data indicating that the determined support option has been carried out and update the plurality of support options accordingly. The third processor 508 is also configured to receive additional information relating to the plurality of support options. This additional information and an updated list of relevant support option is stored in a storage unit 509. Another storage unit 507 stores a list of the implemented support options.

In some embodiments, the first processor 503 may also be configured to receive scheduling data. In these embodiments, the first processor 503 may be configured to combine and analyze this scheduling data with the data received from the sensor arrays.

In some embodiments, the information relating to the plurality of support options relates to an additional support option. This additional support option may be derived from a user input. The process is configured to add this additional support option to the plurality of support options stored by storage unit 509.

In some embodiments, the system 500 may include a device configured to provide contextual information indicating the patient's status (e.g. by collecting vital signs) or relating to the patient's medication schedule. In these embodiments, the third processor 508 is configured to update the plurality of support options based on this information. The first processor 503 and/or second processor 504 may also be configured to determine the preferred support option based on this contextual information.

In some embodiments, support options that have become irrelevant are removed from the plurality of support options stored by the storage unit 509. For example, if a patient is admitted to hospital the support options involving meeting the patient's medical needs at home will no longer be relevant and can be removed from the plurality of support options. The method may involve accessing and extracting information from the patient's electronic medical record to identify a hospital admission. As another example, survey data from the patient may indicate an improvement in mood that means that support options to provide emotional and mental health support are no longer relevant. As another example, the patient may begin receiving more formal support to meet certain healthcare needs (for example, they may become eligible for meal on wheels following a new diagnosis), such that some types of support from the caregiver (e.g., nutrition assistance) may become less relevant.

In some embodiments, the system 500 may learn how to optimize future interactions. This can be based on machine learning algorithms, or rule based methods. For example, the system 500 may be configured to receive user feedback from the patient and/or the caregiver (or multiple caregivers in embodiments involving more than one caregiver). The system may use this information to learn the patient's and/or caregiver's preferences. For example, the feedback may indicate that a specific caregiver is more suited for carrying out a specific support option. In some embodiments, the system may learn to prioritize the support options that the patient appreciates the most.

In some embodiments, the system 500 includes a timer configured to set a predetermined time for certain support options to be completed. After the predetermined time has elapsed, the support options that have become irrelevant are removed from the plurality of support options stored by storage unit 509. Some support options may only be relevant at a certain time of day (e.g., closing or opening blinds, or administering certain medications). Support options that are no longer relevant due to the time of day may be removed from the plurality of support options - at least temporarily.

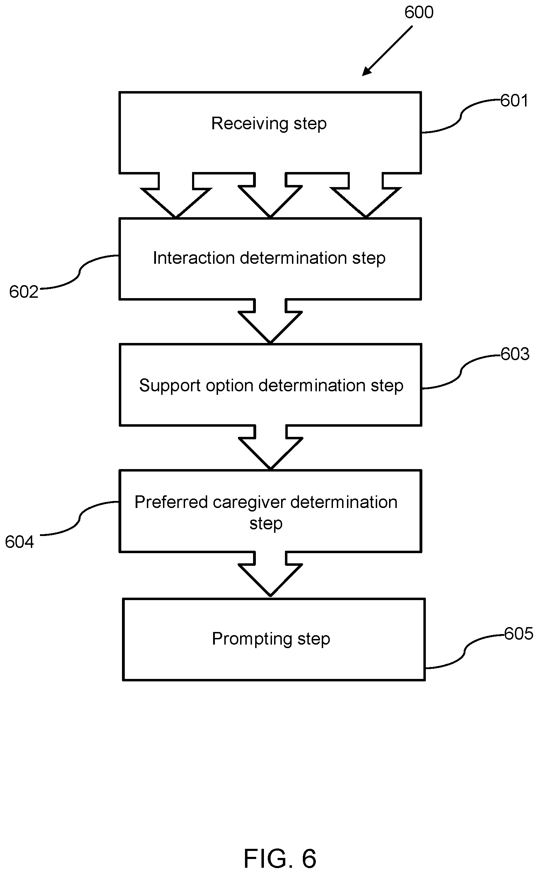

Fig. 6 shows another example of a computer implemented method 600 for providing healthcare support to a patient from a caregiver. The method includes a receiving step 601 comprising receiving data from multiple caregivers. The method also includes an interaction determination step 602 for determining, based on the received data, whether an interaction between each caregiver and the patient is occurring, or when a potential interaction between each caregiver and the patient may occur. This embodiment involves a support option determination step 603 for determining an appropriate support option. In response to determining that multiple interactions are occurring or may occur between the patient and different caregivers over a predetermined time period, a preferred caregiver determination step 604 involves determining a preferred caregiver for providing a determined support option. A prompting step 605 comprises prompting the preferred caregiver to perform the determined support option.

The support option determination step 603 and the preferred caregiver determination step 604 can be performed in any order. The preferred caregiver may be determined by taking into account the determined support option if the support option determination step 603 is carried out before the preferred caregiver determination step 604. Alternatively, the support option determination may take into account the determined preferred caregiver if the preferred caregiver determination step 604 is carried out before the support option determination step 603.

In some embodiments, determining the preferred caregiver comprises processing stored data relating to the availability and skills of each caregiver. For example, the stored data may indicate the availability at different times of each caregiver. The stored data may also indicate which support options each caregiver is willing to provide, and whether they have access to transport. The stored data may be collected using a survey or based on user input. In embodiments involving multiple caregivers the computer implemented method may further comprise sending an update to the plurality of caregivers regarding the support options which have been performed.

In some embodiments, determining the preferred caregiver comprises determining which caregiver is closer to the patient using location sensors. The closest caregiver may be determined to be the preferred caregiver because an interaction is more likely to occur.

The method 600 may further comprise a step for receiving user feedback. The user feedback is provided via a user input, e.g., a graphical user interface. This feedback may be from the patient and/or any of the caregivers. Any of these users may want to give feedback on the system - e.g., patient satisfaction, provider stress, conveniency, etc. This information can be used to optimize future interactions.

For example, some caregivers may require more notice than others in order to provide support options. The feedback from the caregivers may indicate that they prefer providing support options when the determined interaction is expected to occur after a predetermined length of time. Accordingly, the method can be optimized so that these caregivers are only prompted to perform a support option if they can be prompted sufficiently far in advance. On the other hand, some caregivers may prefer to provide more spontaneous support - i.e., in response to an interaction being identified as currently occurring. Therefore, in some embodiments, the determination of the preferred caregiver may be based on how much notice the caregivers prefer.

The inventors have recognized that optimizing future interactions based on user feedback can facilitate a reasonable balance between certainty of the appropriate support option and advance notice for the caregiver. More specifically, in some cases, the further in the future that an interaction is predicted the less certain the appropriate support option is. For example, it can be challenging to predict whether a patient will still need emotional support during an interaction predicted in a few weeks. User feedback can be used to determine how relevant a support option predicted in advance still was by the time the support option was performed. Accordingly, future interactions can be optimized based on feedback indicating which support options are likely to remain relevant for interactions occurring at specific times in the future.

In some embodiments, the feedback may indicate which caregiver the patient prefers to carry out certain support options. For example, the patient may report higher satisfaction when a specific caregiver provides more intimate support options (e.g. hygiene assistance). Accordingly, the method can be optimized so that the determination of the preferred caregiver is based on the patient's caregiver preferences for a specific support option.

In some embodiments, the user feedback may indicate the caregivers' preferences in terms of which support option they perform. For example, some caregivers may prefer to offer emotional support, whereas another will prefer to accompany the patient to medical appointments so that they can better understand the patient's medical journey. These preferences can be taken into account to optimize future interactions.

The inventors have realized that by evaluating satisfaction from both the patients and caregivers support options can be carried out more effectively, thereby facilitating better health outcomes for the patient and decreased caregiver stress.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

In embodiments involving the provision of user input, this can be provided by a graphical user interface. However, the user input can also be provided by other means - e.g., using a keyboard, or by receiving an audio message.

In embodiments, the support option determination step involves determining at least one support option from the plurality of support options. In some embodiments, more than one support option may be determined.

In will be understood that any features of the method explained in relation to embodiments with one caregiver also apply to embodiments including multiple caregivers, and vice versa.

Functions implemented by a processor may be implemented by a single processor or by multiple separate processing units which may together be considered to constitute a "processor". Such processing units may in some cases be remote from each other and communicate with each other in a wired or wireless manner. For example, processors 503 and 504 shown in Fig 5 may be replaced by a single processor configured to carry out the same steps. Likewise, processors 503, 504 and 508 shown in Fig. 5 may be replaced by a single processor configured to carry out the same steps. Similarly, storage units 507 and 509 shown in Fig. 5 may be replaced by a single storage unit.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer implemented method for providing healthcare support, wherein the method comprises carrying out a healthcare support process comprising the steps of:
receiving data from a patient and/or a caregiver;
determining, based on the received data, whether an interaction between the caregiver and the patient is occurring, or when a potential interaction between the caregiver and the patient may occur;
in response to determining that the interaction between the caregiver and the patient is occurring, or when a potential interaction between the caregiver and the patient may occur,
determining a support option from a plurality of support options, and
prompting the caregiver to perform the determined support option.

2. The computer implemented method according to claim 1, wherein the healthcare support process further comprises:
collecting data from the patient using a patient sensor array, and/or
collecting data from the caregiver using a caregiver sensor array.

3. The computer implemented method according to claim 2, wherein the patient sensor array and/or caregiver sensor array is configured to detect communication between the patient and the caregiver,
wherein the step of determining whether interaction between the caregiver and patient is occurring, or when a potential interaction between the caregiver and patient may occur comprises processing the detected communication.

4. The computer implemented method according to claim 2 or claim 3,
wherein the patient sensor array and/or caregiver sensor array comprises a location sensor, and
wherein the step of determining whether interaction between the caregiver and patient is occurring, or when a potential interaction between the caregiver and the patient may occur comprises processing location data received from the location sensor to determine the location of the caregiver and/or patient.

5. The computer implemented method according to any preceding claim,
wherein the data from the patient and/or the caregiver comprises scheduling data, and
wherein the step of determining whether interaction between the caregiver and the patient is occurring, or when a potential interaction between the caregiver and the patient may occur comprises processing the scheduling data received from the patient and/or the caregiver to identify a scheduled meeting between the patient and the caregiver.

6. The computer implemented method according to any preceding claim, wherein the healthcare support process further comprises:
collecting data from the patient using a patient sensor,
collecting data from the caregiver using a caregiver sensor,
determining the distance between the patient and the caregiver based on the data from the patient sensor and the caregiver sensor, and
wherein the step of determining whether interaction between the caregiver and the patient is occurring, or when the next interaction between the caregiver and the patient will occur uses the distance between the patient and the caregiver as an input.

7. The computer implemented method according to any preceding claim, further comprising:
determining whether a user is authorized to act as a caregiver to the patient, and in response to determining that the user is authorized to act as a caregiver, carrying out the healthcare support process of any preceding claim, and optionally
receiving an access code as an input, and determining whether the user is authorized to act as a caregiver to the patient based on the input.

8. The computer implemented method according to any preceding claim,
wherein determining a support option from the plurality of support options comprises processing data received from the patient and/or the caregiver to extract contextual information about the current or potential interaction and using the contextual information to determine a preferred support option, and
wherein prompting the caregiver to perform the determined support option comprises prompting the caregiver to perform the preferred support option.

9. The computer implemented method according to any preceding claim wherein the healthcare support process further comprises:
after prompting the caregiver to perform the determined support option, receiving a user input relating to an additional support option, and
adding the additional support option to the plurality of support options.

10. The computer implemented method according to any preceding claim wherein the healthcare support process further comprises:
receiving user feedback from the patient and/or caregiver as an input,
storing the user feedback, and
optimizing the support provided based on the stored feedback.

11. The computer implemented method according to any preceding claim wherein the method for providing healthcare support is a method for providing medical support, wherein the healthcare support process is a medical support process and the support options are medical support options.

12. The computer implemented method according to any preceding claim, wherein the healthcare support process further comprises:
detecting that the support option has been provided,
in response to detecting that the support has been provided, removing the support option from the plurality of support options and optionally wherein detecting that the support has been provided is based on a user input.

13. The computer implemented method according to any preceding claim, wherein the healthcare support process further comprises:
receiving data from multiple caregivers,
determining, based on the received data, whether an interaction between each caregiver and the patient is occurring, or when a potential interaction between each caregiver and the patient may occur;
in response to determining that multiple interactions are occurring or may occur between the patient and different caregivers over a predetermined time period, determining a preferred caregiver for providing a determined support option,
wherein prompting the caregiver to perform the determined support option comprises prompting the preferred caregiver to perform the determined support option.
and optionally wherein determining the preferred caregiver comprises processing stored data relating to the availability and skills of each caregiver.

14. A system for implementing the computer implemented method of any of claims 1-13, the system comprising:
a processor configured to carry out the method of any preceding claim.

15. A computer program product comprising instructions which, when the program is executed by a computer, cause the computer to carry out the steps of the computer implemented method of any of claims 1-13.
